**Europäisches Patentamt**

**European Patent Office**

**Office européen des brevets**

(19)

(11) Publication number: **0 067 564**
**A2**

# EUROPEAN PATENT APPLICATION

(12)

(21) Application number: **82302652.1**

(22) Date of filing: **24.05.82**

(51) Int. Cl.³: **C 12 N 15/00**

(30) Priority: **23.05.81 JP 78366/81**

(43) Date of publication of application:
**22.12.82 Bulletin 82/51**

(84) Designated Contracting States:
**CH DE FR GB LI NL**

(71) Applicant: **YAMANOUCHI PHARMACEUTICAL CO. LTD.**
No. 5-1 Nihonbashi-Honcho, 2-chome Chuo-ku
Tokyo(JP)

(71) Applicant: **Ogawara, Hiroshi**
2-33-9, Yushima
Bankyo-ku Tokyo(JP)

(72) Inventor: **Ogawara, Hiroshi**
2-33-9, Yushima Bunkyo-ku
Tokyo(JP)

(72) Inventor: **Nakano, Michiko**
4-21-17, Kazino-cho Koganei-shi
Tokyo(JP)

(74) Representative: **Geering, Keith Edwin et al,**
REDDIE & GROSE 16 Theobalds Road
London WC1X 8PL(GB)

(54) Composite plasmids.

(57) A composite plasmid selected from plasmid pMN1 having a molecular weight of 10.0 megadaltons, plasmid pMN2 having a molecular weight of 7.6 megadaltons, and plasmids pMN1' and pMN2' having a molecular weight of 11.3 megadaltons, said composite plasmid being formed by inserting plasmid pSL1 obtained from *Streptomyces lavendulae* into plasmid pCR1 obtained from *Escherichia coli* and having a kanamycin-resistant genetic marker and the following digestion characteristics to restriction enzymes:

(i) pMN1 and pMN2 having one cleavage site to each of EcoRI and SalI, and

(ii) pMN1' and pMN2' having two cleavage sites to each of EcoRI and SalI.

The composite plasmids have kanamycin-resistant genetic markers and hence transformant can be easily selected. The composite plasmids retain the DNA structure of actinomyces plasmid pSL1. In particular, composite plasmid pMN1 retains the greater part of the DNA structure of pSL1. The composite plasmids can effect DNA amplification in the presence of chloramphenicol.

EP 0 067 564 A2

# COMPOSITE PLASMIDS

This invention relates to a composite plasmid obtained from E. coli plasmid pCR1 and an actinomyces (S.lavendulae - see, e.g. merck Index, 7th edition, page 982) plasmid pSL1.

Plasmids are extranuclear genes and are widely used in the field of genetic engineering as vectors for in vitro genetic recombination. The in vitro genetic recombination technique has wide uses such as the growth of useful microorganisms, and the production of useful biologically active materials. Cloning in actinomyces would be useful for investigating antibiotic biosynthesis mechanisms and for obtaining new types of antibiotics, but a cloning system in actinomyces has not yet been established. One of the reasons for this is that suitable vectors with selective markers have not been available. The inventors previously succeeded in isolating plasmid pSL1 from the actinomyces S. lavendulae (see FEMS Microbiol Lett., 9, 111-113 (1980)). The plasmid PSL1 is a potentially useful cloning vector since it has low molecular weight, multi-copies, and only one cleavage site for restriction enzymes such as EcoRI, and SalI, etc.

As the result of further investigations, the invention provides a family of new composite plasmids (termed herein pMN1, pMN2, pMN1', and pMN2') said plasmids being formed by inserting actinomyces plasmid pSL1 into kanamycin-resistant plasmid pCR1 obtained from E. coli.

The invention provides a composite plasmid selected from plasmid pMN1 having a molecular weight of 10.0 megadaltons, plasmid pMN2 having a molecular weight of 7.6 megadaltons, and plasmids pMN1' and pMN2' having a molecular weight of 11.3 megadaltons, said composite plasmid being formed by inserting plasmid pSL1 obtained from Streptomyces lavendulae into plasmid pCR1 obtained from Escherichia coli and having a kanamycin-resistant genetic marker and the following digestion characteristics to restriction enzymes:

(i) pMN1 and pMN2 having one cleavage site to each of EcoRI and SalI, and

(ii) pMN1' and pMN2' having two cleavage sites to each of EcoRI and SalI.

The composite plasmids pMN1, pMN2, pMN1', and pMN2' of this invention can provide a new method of molecular cloning and possess the following properties:

1. The composite plasmids have kanamycin-resistant genetic markers and hence transformant can be easily selected.

2. The composite plasmids retain the DNA structure of actinomyces plasmid pSL1. In particular, composite plasmid pMN1 retains the greater part of the DNA structure of pSL1.

3. Composite plasmids pMN1 and pMN2 have one cleavage site to restriction enzymes EcoRI and SalI.

4. The composite plasmids can effect DNA amplification in the presence of chloramphenicol.

**0067564**

Since composite plasmids pMN1, pMN2, pMN1' and pMN2' stem from the plasmid of E. coli and the plasmid of actinomyces as described above, the invention provides for a shuttle vector capability by subjection of composite plasmid pMN1, pMN2, pMN1' or pMN2' to cloning in E. coli to amplify the gene in E. coli, return of the gene to the actinomyces, and subjection of the gene to genetic cloning in the actinomyces.

The general procedures for the formation and isolation of composite plasmids pMN1, pMN2, pMN1' and pMN2' of this invention may be as follows:

(1)   Cleavage and recombination of plasmid DNA by restriction enzymes

The cleavage of plasmid DNA with restriction enzymes (EcoRI and SalI) is performed at 37°C according to the assay conditions set out   in the directions for use of the restriction enzymes.   The cleavage fragments are mixed and precipitated with cold ethanol. The precipitates are centrifuged, dried in vacuo, and dissolved in a ligation mixture (66 mM Tris-HCl, pH 7.6, 6.6mM MgCl$_2$, 10 mM dithiothreitol, and 0.4 mM ATP).  Then, T4 ligase is added to the mixture and the resultant mixture is incubated at 12°C for 40 hours.  The mixture is reprecipitated with cold ethanol and the precipitates are resuspended in TEN buffer (10 mM Tris-HCl, 50 mM NaCl, and 1 mN EDTA, pH 7.6).

(2)   Transformation of E. coli.

The transformation is performed according to the method described in Norgard et al; "Gene", 3, 279-292(1978).

(3)   Colony hybridization.

Colony hybridization is performed by a modification of the hybrid formation method described in Grustein and Hogness: "Proc. Natl. Acad. Sci. USA", 72, 3961-3965(1975). The kanamycin-resistant transformant of E. coli is transferred onto a nitrocellulose filter placed on an agar plate containing kanamycin (50 µg/ml) (the filter being washed twice with boilling water and treated in an autoclave for 10 minutes before being placed on the agar plate).   Then the filter is transferred onto the same agar plate containing chloramphenicol (170 µg/ml) and further incubated overnight at 37°C.  The filter is withdrawn from the agar plate and placed on Whatman 3MM papers presoaked with 0.5 M NaOH for 7 minutes to effect lysis and DNA denaturation. The filter is washed with 1 M Tris-HCl of pH 7.4 for 2 minutes and then 0.5 M Tris-HCl-1.5 M NaCl of pH 7.4 for 4 minutes and dried. After further washing with chloroform and then 0.3 M NaCl, the filter is treated in a vacuum oven at $80^{\circ}C$ for 2 hours.

The hybrid forming treatment is performed with a filter in a hybrid forming buffer (50% formamide-6 X SSC, 10 mM sodium pyrophosphate, 0.02% polyvinyl pyrrolidone, 0.02% bovine serum albumin, 0.02% Ficoll, 0.5% sodium lauryl sulfate, and 20 $\mu$g/ml of denatured salmon sperm DNA) containing $^{32}$P-labeled pSL1 DNA (Rigby et al: "J. Mol. Biol."; 98, 503-517(1977)) at $52^{\circ}C$ for 36 hours. Thereafter, the filter is washed and autoradiographed for 16 hours at $-70^{\circ}C$.

(4) Screening of composite plasmid.

Transformants having the composite plasmid are first selected by kanamycin resistance coded by pCR1 and then selected by the colony hybridization technique using labelled pSL1. The presence of composite plasmid of pCR1 and pSL1 in the clones hybridized with labeled pSL1 can thus be distinguished

(5) Isolation of composite plasmid.

The isolation of the composite plasmid is performed by the microscale technique described in Klein, Selsing, and Wells: "Plasmid"; 3, 88-91(1980). This microscale technique involves cell lysis with phenol, centrifugation, phenol extraction, ethanol precipitation, RNase digestion, etc., and is suitable for isolating the composite plasmid for restriction enzyme analysis.

There are two kinds of composite plasmid pMN1' (Type I) and pMN2' (Type II), according to the different configurations of the pSL1 combination, and these configurations can be differentiated by the digestion pattern of plasmid DNA with EcoRI.   The configurations shown in the foregoing schematic programme of the construction of composite plasmid are evident from the fact that in the case of Type I cleavage with EcoRI gives two fragments of 10 Md (megadalton) and 1.3 Md and that in the case of Type II the cleavage gives two fragments of 7.6 Md and 3.7 Md.

6) Isolation of composite plasmids pMN1 and pMN2.

The composite plasmids of Type I and Type II each has two cleavage sites by EcoRI and SalI and each of the cleavage sites of EcoRI and SalI can be deleted individually by the following operation:

The composite plasmids of Type I and Type II are cleaved by the restriction enzyme EcoRI and subjected to agarose electrophoresis.   The resulting high molecular weight fragment (the 10 Md fragment in the case of Type I and the 7.6 Md fragment in the case of Type II) is extracted from the gel and ligated with T4 ligase.   E. coli 600 is infected with the ligated mixture, kanamycin resistant clones are selected, and the DNA plasmids (pMN1 and pMN2) are isolated from the transformants.   The isolated plasmids pMN1 and pMN2 have the following properties:

i)  Molecular weight - pMN1:  10.0 Md;  pMN2: 7.6 Md.

ii) One cleavage site to EcoRI and SalI.

iii) Double digestion by different restriction enzymes of EcoRI and SalI. Thus:

a) Plasmid pMNl gives two fragments of 7.5 Md and 2.5 Md; the former corresponds to the EcoRI and SalI cleavage fragment of pCRI, and the latter corresponds to the cleavage fragment of pSL1.

b) Plasmid pMN2 gives two fragments of 7.5 Md and 0.1 Md; Their assignment is as in a).

iv) The fragment digested by EcoRI does not contain kanamycin resistant genes (that is, plasmids pMN1 and pMN2 contain kanamycin resistant genes).

The invention will be further explained by the following Example. Plamid pSL1 used in the Example is the plasmid prepared by the method described in "FEMS. Microbiol. Lett."; 9, 111-113(1980) and plasmid pCRI is a known one - see "Molecular and General Genetics", 145, 155-156(1976). The host E. coli C600 (see "Gene", Jan.1980, p. 138), the restriction enzymes (EcoRI and SalI), and the ligase (T4 ligase) used in the Example are all known and commercially available

## EXAMPLE

(1) Restriction and recombination of pCR1 and pSL1:

The plasmids pCR1 (0.4 µg) and pSL1 (0.2 µg), purified by subjection twice to caesium chloride-ethidium bromide density gradient centrifugation, were completely digested with restriction enzyme SalI (made by Takara Shuzo K.K.). The reaction was stopped by heating to 65°C for 10 minutes; after adding 2-3 times by volume of cold ethanol (-20°C) to the reaction mixture, the mixture was allowed to stand for 15 minutes at -70°C, and the precipitates thus formed were recovered by centrifugation for 20 minutes at 16,000 r. p. m.   After suspending the precipitates in 150 $\mu$l of a ligase reaction mixture (66 mM Tris-HCl, pH 7.6, 6.6 mM MgCl$_2$, 10 mM dithiothritol, and 0.4 mM ATP), T4 ligase was added thereto and the mixture was reacted for 40 hours at 12$^o$C.

The reaction mixture was precipitated again with cold ethanol and DNA was resuspended in 200 $\mu$l of TEN buffer (10 mM Tris-HCl, 50 mM NaCl, and 1mM EDTA, pH 7.6).  The DNA solution after the ligase reaction was subjected to a 0.7% agarose gel electrophoresis and after dyeing with ethidium bromide (1 $\mu$g/ml), the portion having a mobility lower than that of pCR1 linear DNA was cut by means of a razor blade under ultraviolet light of 350 n. m. and injected into 2 ml of a TEN buffer by means of an injector.  After allowing to stand overnight at 4$^o$C, the mixture was frozen at -70$^o$C for 2 hours and after fusing and then centrifuging the mixture for 30 minutes at 30,000 x g, the supernatant was filtered by means of a micropore filter (HAWP).  After extracting    remaining ethidium bromide with isoamyl alcohol, the residue was dialyzed against 0.1 x SSC (15 mM NaCl and 1.5 mM sodium citrate)-1 mM EDTA (pH 7.5).

(2)  Trasformation of E. coli:

E. coli C600 thi thr leu lac r$^-$ m$^-$ was innoculated into 10 ml of Penassay broth (Difco) and after             shaking cultivation overnight at 37$^o$C, 0.1 ml of a seed  culture    was added to 10 ml of the same culture medium.  After further incubation

for 2.5 hours, cells were collected and washed twice with 0.1 M NaCl-5 mM MgCl$_2$-5 mM Tris-HCl (pH 7.6).  The washed cells were suspended in 4 ml of 100 mM CaCl$_2$-250 mM KCl- 5 mM MgCl$_2$-5 mM Tris-HCl (pH 7.6) and after allowing to stand

for 25 minutes at 0°C, the suspension was centrifuged again. The cells thus recovered were resuspended in 0.6 ml of      buffer having the same composition as above and after adding thereto 400 μl of DNA extracted previously from an agarose gel, the mixture was allowed to stand for 60 minutes at 0°C. Then, after treating the mixture for 2 minutes at 42°C, the mixture was added to 10 ml of Penassay broth, and after

shaking cultivation for one hour at 37°C the cultured product was seeded onto an agar culture medium incorporating 50 μg/ml of
kanamycin.

   (3)   Colony hybridization:

   A Schleicher & Schull nitrocellulose filter (13   x 9.5 cm ) was washed twice in boiling water, inserted between Whatman 3MM

papers, and subjected to autoclave  sterilization for 10 minutes.

After drying, the filter was superposed on an agar culture

medium incorporating 50 μg/ml of kanamycin and the kanamycin-

resistant strain (having pCR1) isolated in foregoing step (2) was replicated by means of a toothpick.  After cultivating overnight at 37°C, the filter having bacteria or cells was transferred onto an agar culture medium incorporating 50 μg/ml of kanamycin and

170 μg/ml of chloramphenicol and the cells were further cultured

overnight at 37°C to amplify plasmids.

   Four 3MM papers previously impregnated with 0.5 N NaOH were laminated and the nitrocellulose filter was placed thereon with the cell-carrying surface at the upper side and treated for 7 minutes.  Similarly, the filter was treated twice/with 1 M Tris-HCl (pH 7.4) for 2 minutes and further with 1.5 M NaCl-0.5 M Tris-HCl (pH 7.4) for 4 minutes.  After washing the filter with chloroform and then 0.3 M NaCl, the filter was dried and kept in vacuo for 2 hours at 80°C. The filter was used for hybridization.

Plasmid pSL 1 labeled with $^{32}$P was prepared as follows. After heating 24 $\mu$l of a reaction mixture containing 7 $\mu$l of $\alpha$-$^{32}$P-dATP (10 mCi/ml), 2.5 $\mu$l of 10 x buffer (500 mM Tris-HCl, pH 7.8, 50 mM MgCl$_2$, 100 mM mercaptoethanol, and 1 mg/ml gelatin), 5 $\mu$l of 25 $\mu$M dTTP, dGTP, dCTP, 8.5 $\mu$l of pSL1 DNA, and 1 $\mu$l of DNase (50 $\mu$g/ml) for 15 minutes at 37°C, 1 $\mu$l of DNA polymerase was added thereto and they were cultured for 2 hours at 15°C. To the culture medium were added 75 $\mu$l of 30 mM Tris-HCl (pH 8.0)-10 mM EDTA and 10 $\mu$l of 2 mg/ml tRNA and then proteins were denatured and removed with the same volume of chloroform/isoamyl alcohol (24 : 1). To the aqueous layer formed was added 10 $\mu$l of 1.5 M sodium acetate solution, DNA was precipitated with 300 $\mu$l of cold ethanol, and after centrifuging, the precipitates thus recovered were dissolved in 100 $\mu$l of 10 mM Tris-HCl (pH 8.0)-1 mM EDTA. By this method, about 5 x 10$^7$ cpm of $^{32}$P count was recovered in the DNA fraction. To the DNA solution were added 36 $\mu$l of 10 mg/ml salmon sperm DNA and 0.5 ml of water and the mixture was denatured for 5 minutes at 100°C and chilled, and used as the probe for the hybridization.

The nitrocellulose filter adsorbed with DNA was prehybridized for 4 hours at 52°C with 6 x SSC, 0.02% PVP (polyvinylpyrrolidone)-0.02% BSA (albumin)-0.02% Ficoll, and 20 $\mu$g/ml tRNA-50% formamide. After pre-hybridization, 200 $\mu$l of a solution of labelled pSL1 was added to 10 ml of a hybridization buffer (6 x SSC, 0.02% PVP-BSA-Ficoll, 0.5% formamide, and 10 mM sodium pyrophosphate), the filter was placed in the buffer and they were allowed to stand for 36 hours at 52°C in a sealed plastics bag. After washing the filter with 6 x SSC, 0.02% PVP-BSA-Ficoll, the filter was treated with the same solution for one hour at 52°C; the same operation was repeated with 6 x SSC,        the filter was finally washed twice with 2 x SSC and after heating to 52°C for 30 minutes, the filter was dried. The filter was then wrapped, brought into close contact with a Fuji X-ray film, and           mounted in a cassette with a Du Pont intensifying screen and allowed to stand for 16 hours at -70°C.

After development, 3 strains of strongly blackened clones were obtained. They were considered to have pSL1.

(4) Confirmation of direction   of combination of hybrid plasmid (pMN1' or pMN2') of pCR1 and pSL1

Plasmid was isolated from the foregoing three strains which were considered to have the hybrid plasmid (pMN1' or pMN2') of pCR1 and pSL1 and the direction   of the combination of pCR1 and pSL1 was determined.

After cultivating overnight the 3 strains in Penassay  broth at 37°C, 1 ml of the cultured solution was transferred to 10 ml of the same culture medium and shaking cultivation was performed again for 1.5 hours. Then, chloramphenicol was added to   170 $\mu$g/ml and after cultivating for 8 hours, the cells were collected. The mycellia were   suspended in 0.5 ml of 50 mM Tris-HCl (pH 8), the suspension was placed in an Eppendorf tube and after adding thereto 0.05 ml of 10 mg/ml lysozyme, the mixture was allowed to stand for 15 minutes at room temperature to perform lysis. Then, 0.5 ml of phenol previously saturated with 50 mM Tris-HCl (pH 8.0) was added to the suspension and mixed by thrice turning over the tube. After centrifuging for 5 minutes at 6,000 r. p. m., 0.3 ml of phenol was added again to the aqueous layer and after shaking vigorously, the aqueous layer was recovered and the extraction of phenol was performed 2-3 times with 1.4 ml of chloroform. DNA was precipitated with 2 times by volume of cold ethanol and the precipitates were suspended in 100 $\mu$l of water, to give a sample of plasmid DNA.

Restriction enzyme treatment was performed at 37°C by adding restriction enzyme and 10 $\mu$l of 1 mg/ml RNase A (the solution was previously heated to 90°C for 10 minutes to inactivate DNase existing in the mixture) to 10 $\mu$l of the sample. From the result of 0.7% agarose electrophoresis, it was shown that the plasmid of three kinds of strains had pCR1 and pSL1 combined in the direction shown in the above-described schematic programme, that is, was pMN1' (Type I).

(5) Preparation of pMN1

One of two EcoRI and SalI sites of the hybrid plasmid confirmed in step (4) was deleted as follows.

After completely digesting 120 $\mu$l of the hybrid plasmid pMN1' with EcoRI, the digested plasmid was subjected to 0.7% agarose gel electrophoresis, and dyed with ethidium bromide. Thereafter, one of two bands thus obtained and having the lower mobility was cut by a razor blade and DNA was extracted in the manner described before. The extracted DNA was precipitated with cold ethanol and after dissolving the precipitates in 200 $\mu$l of a ligation buffer, 1 $\mu$l of T4 ligase was added thereto and reacted for 40 hours

0067564

at 12<sup>o</sup>C.  <u>E. coli</u> C600 was transformed with the ligase reaction mixture by the method previously described to isolate the kanamycin resistant strain.  The plasmid DNA of the kanamycin resistant strain was determined by  restriction enzyme treatment, etc., and was named pMN1.

BAD ORIGINAL

CLAIMS:

1. A composite plasmid selected from plasmid pMN1 having a molecular weight of 10.0 megadaltons, plasmid pMN2 having a molecular weight of 7.6 megadaltons, and plasmids pMN1' and pMN2' having a molecular weight of 11.3 megadaltons, said composite plasmid being formed by inserting plasmid pSL1 obtained from Streptomyces lavendulae into plasmid pCR1 obtained from Escherichia coli and having a kanamycin-resistant genetic marker and the following digestion characteristics to restriction enzymes:

(i) pMN1 and pMN2 having one cleavage site to each of EcoRI and SalI, and

(ii) pMN1' and pMN2' having two cleavage sites to each of EcoRI and SalI.

2. A method of preparing composite plasmid pMN1' or pMN2' which comprises enzymatic restriction of plasmids pCR1 and pSL1, and reaction of the cleavage fragments in a ligase reaction mixture.

3. A method of preparing a composite plasmid pMN1 which comprises enzymatic restriction of composite plasmid pMN1' and separation of the 10 Md cleavage fragment.

4. A method of preparing a composite plasmid pMN2 which comprises enzymatic restriction of composite plasmid pMN2' and separation of the 7.6 Md cleavage fragment.